# EUROPÄISCHE PATENTSCHRIFT

(11) **EP 1 379 220 B1**
(45) Veröffentlichungstag und Bekanntmachung des Hinweises auf die Patenterteilung: **29.12.2004**
(21) Anmeldenummer: 02776507.2
(22) Anmeldetag: 27.05.2002
(51) Int. Cl.: A61K 9/00

(54) **INHALATIONSKAPSELN**
INHALATION CAPSULES
CAPSULES POUR INHALATION

(30) Priorität: 01.06.2001 DE 10126924
(43) Veröffentlichungstag der Anmeldung: 14.01.2004
(60) Teilanmeldung: 04026411.1
(73) Patentinhaber: Boehringer Ingelheim Pharma GmbH & Co.KG, 55216 Ingelheim am Rhein (DE)
(72) Erfinder: HOCHRAINER, Dieter, 57392 Schmallenberg (DE); BECHTOLD-PETERS, Karoline, 88400 Biberach/Rissegg (DE); WALZ, Michael, 55411 Bingen (DE); TRUNK, Michael, 55218 Ingelheim (DE)
(86) Internationale Anmeldenummer: PCT/EP2002/005600
(87) Internationale Veröffentlichungsnummer: WO 2002/098874

(56) Entgegenhaltungen:
- EP-A- 0 418 716
- EP-A- 1 072 258

## Beschreibung

Die Erfindung betrifft Inhalationskapseln (Inhaletten) aus spezifischen Kapselmaterialien mit reduziertem Feuchtegehalt, die den Wirkstoff Tiotropium in Form pulverförmiger Zubereitungen enthalten und durch eine erhöhte Stabilität gekennzeichnet sind.

### Hintergrund der Erfindung

Tiotropiumbromid ist aus der Europäischen Patentanmeldung EP 418 716 A1 bekannt und weist die folgende chemische Struktur auf:

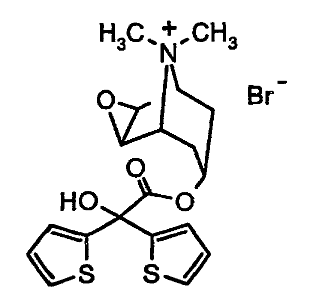

Tiotropiumbromid stellt ein hoch wirksames Anticholinergikum mit langanhaltender Wirkdauer dar, welches zur Therapie von Atemwegserkrankungen, insbesondere von COPD (chronic obstructive pulmonary disease = chronisch obstruktive Lungenerkrankung) und Asthma Verwendung finden kann. Unter Tiotropium ist das freie Ammoniumkation zu verstehen.

Bei der Behandlung vorstehender Erkrankungen bietet sich die inhalative Applikation des Wirkstoffs an. Neben der inhalativen Applikation von broncholytisch wirksamen Verbindungen in Form von Dosieraerosolen und Lösungen kommt der inhalativen Applikation dieser Arzneistoffe in Form von wirkstoffhaltigen Pulvern besondere Bedeutung zu.

Bei Wirkstoffen, die eine besonders hohe Wirksamkeit aufweisen, sind pro Einzeldosis zur Erzielung des therapeutisch erwünschten Effekts nur geringe Mengen des Wirkstoffs erforderlich. In solchen Fällen ist es notwendig, zur Herstellung des Inhalationspulvers den Wirkstoff mit geeigneten Hilfsstoffen zu verdünnen. Bei durch eine hohe Wirksamkeit gekennzeichneten Wirkstoffen ist es zur Gewährleistung eines bei der Applikation stets reproduzierbar gleichbleibenden Anteils besonders wesentlich, das Arzneimittel in einer Form bereitzustellen, die durch ein hohes Maß an Stabilität gekennzeichnet, ist. Wird dieses hohe Maß an Stabilität nicht erzielt, so kann eine gleichbleibende Dosierung des Wirkstoffs nicht sichergestellt werden.

Es ist Aufgabe der Erfindung, eine ein Tiotropium-haltiges Inhalationspulver enthaltende Inhalationskapsel bereitzustellen, die ein ausreichendes Maß an Stabilität des Wirkstoffs gewährleistet.
Es ist ferner Aufgabe der Erfindung eine Inhalationskapsel bereitzustellen, die aufgrund ihrer Stabilität die Freisetzung des Wirkstoffs mit hoher Dosiergenauigkeit (betreffend die pro Inhalationskapsel herstellerseitig abgefüllte Menge an Wirkstoff und Pulvermischung wie auch die pro Inhalationskapsel durch den Inhalationsvorgang ausgebrachte und lungengängige Wirkstoffmenge) gewährleistet. Ferner ist es Aufgabe der vorliegenden Erfindung eine Inhafationskapsel bereitzustellen, die die Applikation des Wirkstoffs bei gutem Entleerungsverhalten der lnhaiationskapsel ermöglicht.
Eine weitere Aufgabe der vorliegenden Erfindung besteht in der Bereitstellung von Inhalationskapseln, die bei hoher Stabilität und geringer Brüchigkeit ein gutes Lochungsverhalten aufweisen und dadurch problemlos in zur Applikation von Inhaletten geigneten lnhalationsgeräten zur Anwendung gelangen können.

### Detaillierte Beschreibung der Erfindung

Überraschenderweise wurde gefunden, daß die eingangs genannten Aufgaben durch die nachstehend beschriebenen, erfindungsgemäßen Inhalationskapseln (Inhaletten) gelöst werden.

Bei den erfindungsgemäßen Inhalationskapseln (Inhaletten) handelt es sich um Kapseln, die als Inhalationspulver Tiotropium im Gemisch mit einem physiologisch unbedenklichen Hilfsstoff enthalten, dadurch gekennzeichnet, daß das Kapselmaterial einen reduzierten Feuchtegehalt aufweist.

Der Begriff des reduzierten Feuchtegehalts ist im Rahmen der vorliegenden Erfindung definiert als äquivalent zu der Bezeichnung einer TEWS-Feuchte von weniger als 15%.

Unter dem Begriff TEWS-Feuchte wird im Rahmen der vorliegenden Erfindung die Feuchte verstanden, die mittels des Feuchtemeßgerät MW 2200 der Firma TEWS bestimmt werden kann. Die Meßmethode ist eine indirekte Methode der Wasserbestimmung. Es werden vom Wassergehalt abgeleitete Wirkungen (Mikrowellenabsorbtion durch das im Produkt enthaltene Wasser) gemessen und als Mikrowellenwert angezeigt. Zur Ermittlung des Wassergehaltes in Gewichtsprozent, ist eine Kalibrierung des Gerätes unter Verwendung von Kalibrierproben notwendig. Die daraus resultierende Kalibrierkurve wird bei nachfolgenden Messungen vom Gerät zur Umrechnungen herangezogen. Der Feuchtewert wird in % angezeigt und abgespeichert.

Zur Kalibrierung des TEWS-Geräts kann wie im Rahmen der vorliegenden Erfindung beispielsweise ein Halogentrockner Verwendung finden. Aufgrund der Kalibrierung des TEWS-Geräts mittels eines Halogentrockners ist im Rahmen der vorliegenden Erfindung der Begriff der TEWS-Feuchte als äquivalent zum Begriff der Halogentrockner Feuchte anzusehen. Beispielsweise entsprechen im Rahmen der vorliegenden Erfindung 15% TEWS-Feuchte einer Halogentrockner-Feuchte von ebenfalls 15%. Während das TEWS-Gerät funktionsbedingt eine Relativmethode zur Wasserbestimmung darstellt, liefert der Halogentrockner Absolutwerte für die Kapselfeuchte. Der Wassergehalt wird beim Halogentrockner durch Gewichtsverlust bestimmt. Die Kapseln werden erhitzt, wobei das Wasser austritt. Die Trocknung der Kapseln wird bis zur Gewichtskonstanz durchgeführt und dann abgelesen. Die Massendifferenz zwischen Ausgangs- und Endgewicht (in Gramm) stellt den Wassergehalt der Kapseln dar und kann in Gewichtsprozent umgerechnet werden. Das TEWS-Gerät vergleicht beim Messen des Wassergehaltes nur die Meßkurven der aktuellen Kapseln mit internen Eichkurven. Diese Eichkurven werden mit Kapseln definierten Wassergehalts aufgenommen, deren absoluter Wassergehalt vorher mit der Halogentrocknermethode ermittelt worden ist.
Auf diese Weise wird für die Relativmethode TEWS der Bezug zu absoluten Wassergehalten mit Hilfe der Halogentrocknermethode hergestellt.

Erfindungsgemäß bevorzugte Inhalationskapseln weisen eine TEWS- oder Halogenirockner-Feuchte von weniger als 12%, besonders bevorzugt von ≤ 10% auf.

Unter Kapselmaterial wird im Rahmen der vorliegenden Erfindung das Material verstanden, aus dem die Hülle der Inhalationskapsel gefertigt ist. Das Kapselmaterial ist erfindungsgemäß ausgewählt aus der Gruppe bestehend aus Gelatine, Cellulosederivaten, Stärke, Stärkederivaten, Chitosan und synthetischen Kunststoffen.

Wird Gelatine als Kapselmaterial verwendet, so kann diese im Gemisch mit anderen Zusätzen ausgewählt aus der Gruppe bestehend aus Polyethylenglycol (PEG), bevorzugt PEG 3350, Glycerol, Sorbitol, Propylenglycol. PEO-PPO-Blockcopolymeren und anderen Polyalkoholen und Polyethern zum Einsatz kommen. Besonders bevorzugt wird im Rahmen der vorliegenden Erfindung Gelatine im Gemisch mit PEG, bevorzugt PEG 3350, verwendet. Besonders bevorzugt enthält eine erfindungsgemäße Gelatine-Kapsel PEG in einem Anteil von 1-10% (Gew-%), bevorzugt 3-8 %. Besonders bevorzugte Gelatine-Kapseln enthalten PEG in einem Anteil von 4-6%, wobei ein PEG-Anteil von etwa 5% erfindungsgemäß höchstbevorzugt ist.
Im Falle Gelatine-haltiger Kapselmaterialien weisen die erfindungsgemäßen Kapseln vorzugsweise eine TEWS- oder Halogentrockner-Feuchte von weniger als 12%, besonders bevorzugt von ≤10% auf.

Werden Cellulosederivate als Kapselmaterial verwendet, so ist die Verwendung von Hydroxypropylmethylcellulose, Hydroxypropylcellulose, Methylcellulose, Hydroxymethylcellulose und Hydroxyethylcellulose bevorzugt. Besonders bevorzugt wird in diesem Fall Hydroxypropylmethylcellulose (HPMC), besonders bevorzugt HPMC 2910 als Kapselmaterial eingesetzt. Im Falle der Verwendung von Cellulosederivaten als Kapselmaterialien liegt der Grad der TEWS- oder Halogentrockner-Feuchte vorzugsweise bei weniger als 8%, besonders bevorzugt bei weniger als 5%. Höchst bevorzugt werden Inhalationskapseln aus Cellulosederivaten vor Befüllung mit dem Tiotropiumhaltigen Inhalationspulver auf eine TEWS- oder Halogentrockner-Feuchte von weniger als 4 %, besonders bevorzugt weniger als 2% getrocknet.

Werden als Kapselmaterial synthetische Kunststoffe eingesetzt, so sind diese erfindungsgemäß bevorzugt ausgewählt aus der Gruppe bestehend aus Polyethylen Polycarbonat, Polyester, Polypropylen und Polyethylenterephthalat. Besonders bevorzugt sind als synthetische Kunststoffmaterialien für die erfindungsgemäßen Inhalationskapseln Polyethylen, Polycarbonat oder Polyethylenterephthalat. Wird Polyethylen als eines der erfindungsgemäß besonders bevorzugten Kapselmaterialien verwendet, gelangt vorzugsweise Polyethylen mit einer Dichte zwischen 900 und 1000 kg/m³, bevorzugt von 940 - 980 kg/m³, besonders bevorzugt von 960 kg/m³ (high-density Polyethylen) zur Anwendung. Im Falle der Verwendung von synthetischen Kunststoffen als Kapselmaterialien kann der Grad der TEWSoder Halogentrockner-Feuchte gegebenenfalls bei weniger als 3%, gegebenenfalls weniger als 1% liegen.

Die erfindungsgemäßen Inhalationskapseln werden nach Bereitstellung der leeren Kapseln in einer der vorstehenden genannten Ausführungen mit Tiotropium-haltigen Inhalationspulver befüllt. Diese Befüllung kann nach im Stand der Technik bekannten Verfahren erfolgen. Die Herstellung der zur Befüllung einzusetzenden leeren inhalationskapseln kann ebenfalls nach im Stand der Technik bekannten Vorgehensweisen erfolgen. Beispielsweise seien als mögliche Herstellungsverfahren die im Stand der Technik bekannten Prozesse des Tauchverfahrens, Blasdruckverfahrens, Spritzgußverfahrens, Extrusionsverfahrens und Taufziehverfahrens genannt.

Erfindungswesentlich ist bei der Herstellung der erfindungsgemäßen Inhalationskapseln, daß, sofern das Kapselmaterial vor Befüllung mit dem wirkstoffhaltigen Inhalationspulver nicht bereits aufgrund entsprechender Lagerung oder Herstellung mit ausreichend reduziertem Feuchtegehalt zur Verfügung steht, eine Trocknung der leeren Kapseln erfolgt. Diese Trocknung wird solange durchgeführt, bis ein Feuchtigkeitsgrad erreicht ist, wie er der erfindungsgemäßen Spezifikation von maximal 15% TEWS- oder Halogentrockner-Feuchte entspricht.

Im Rahmen der vorliegenden Erfindung ist die Bezeichnung Inhalationskapsel als mit der Bezeichnung Inhalette gleichbedeutend anzusehen.

Ein weiterer Aspekt der vorliegenden Erfindung betrifft die Verwendung von. Kapseln die durch eine TEWS- oder Halogentrockner-Feuchte von weniger als 15% gekennzeichnet sind und aus den vorstehend genannten Kapselmaterialien bestehen können, zur Herstellung von Inhaletten (Inhalationskapseln), die Tiotropium-haltiges Inhalationspulver enthalten. Im Rahmen der vorliegenden Erfindung ist die Bezeichnung Kapsel als Bezugnahme auf leere, also noch kein Inhalationspulver enthaltende Inhalationskapseln anzusehen.

Erfindungsgemäß bevorzugt sind Inhalationskapseln, die Inhalationspulver mit einem Gehalt von 0,001 bis 2 % Tiotropium enthalten. Besonders bevorzugt sind Inhalationskapseln, die Inhalationspulver mit 0,04 bis 0,8%, bevorzugt 0,08 bis 0,64%, besonders bevorzugt 0,16 bis 0,4% Tiotropium enthalten. Die bezüglich des Anteils von Tiotropium im Rahmen der vorliegenden Erfindung gemachten prozentualen Angaben entsprechen Gewichtsprozent bezogen auf die Gesamtmenge an Inhalationspulver.

Unter Tiotropium ist das freie Ammoniumkation zu verstehen. Als Gegenion (Anion) kommen Chlorid, Bromid, Iodid, Methansulfonat, para-Toluolsutfonat oder Methylsulfat in Betracht. Von diesen Anionen ist das Bromid bevorzugt. Dementsprechend betrifft die vorliegende Erfindung bevorzugt Inhaletten, die Inhalationspulver enthalten, welche durch einen Gehalt von 0,0012 - 2,41% Tiotropiumbromid gekennzeichnet sind.

Erfindungsgemäß von besonderem Interesse werden Inhalationspulver eingesetzt, die zwischen 0,048 und 0,96%, bevorzugt 0,096 bis 0,77%, besonders bevorzugt 0,19 bis 0,48% Tiotropiumbromid enthalten.

Das in den erfindungsgemäßen Inhaletten enthaltene Inhalationspulver kann das erfindungsgemäß bevorzugt enthaltene Tiotropiumbromid in Form seiner Hydrate enthalten. Besonders bevorzugt wird kristallines Tiotropiumbromid-monohydrat eingesetzt. Dementsprechend betrifft die vorliegende Erfindung Inhaletten, die Inhalationspulver mit einem Gehalt von zwischen 0,0012 und 2,5% an kristallinem Tiotropiumbromid-monohydrat enthalten. Erfindungsgemäß von besonderem Interesse sind Inhaletten, die Inhalationspulver mit einem Gehalt von 0,05 und 1%, bevorzugt 0,1 bis 0,8%, besonders bevorzugt 0,2 bis 0,5% kristallinem Tiotropiumbromid-monohydrat enthalten.
Im Rahmen der vorliegenden Erfindung ist eine Bezugnahme auf die Bezeichnung Tiotropiumbromid-monohydrat vorzugsweise als Bezugnahme auf jenes kristalline Tiotropiumbromid-monohydrat zu verstehen, welches gemäß der im experimentellen Teil detailliert erläuterten synthetischen Vorgehensweise erhältlich ist.

Die in die erfindungsgemäßen Inhalationskapseln (Inhaletten) eingesetzten Inhalationspulver enthalten neben dem Wirkstoff wenigstens einen Hilfsstoff. Dieser kann aus einer bezüglich der mittleren Teilchengröße der Hilfsstoffpartikel einheitlichen Hiffsstofffraktion (beispielsweise 15-80µm) bestehen oder gegebenenfalls ein Gemisch von gröberem Hilfsstoff mit einer mittleren Teilchengröße von 15 bis 80µm und feinerem Hitfststoff mit einer mittleren Teilchengröße von 1 bis 9 µm darstellen. Werden Hilfststoffgemische aus gröberen und feineren Hilfststoffanteilen eingesetzt, beträgt der Anteil von feinerem Hilfsstoff an der Gesamthilfsstoffmenge vorzugsweise 1 bis 20%.
Besonders bevorzugt enthalten die erfindungsgemäßen Inhalationskapseln, sofern sie aus einem Gemisch aus gröberen mit feineren Hilfsstofffraktionen bestehen, gröberen Hilfsstoff mit einer mittleren Teilchengröße von 17 bis 50µm, besonders bevorzugt von 20 bis 30µm und feinerem Hilfststoff mit einer mittleren Teilchengröße von 2 bis 8µm, besonders bevorzugt von 3 bis 7µm besteht. Dabei wird unter der mittleren Teilchengröße im hier verwendeten Sinne der 50%-Wert aus der Volumenverteilung gemessen mit einem Laserdiffraktometer nach der Trockendispersionsmethode verstanden. Bevorzugt werden Inhalationspulver zur Herstellung der erfindungsgemäßen Inhaletten verwendet, bei denen der Anteil von feinerem Hilfsstoff an der Gesamthiffsstoffmenge 3 bis 15%, besonders bevorzugt 5 bis 10% beträgt. Bei den im Rahmen der vorliegenden Erfindung genannten prozentualen Angaben, handelt es sich stets um Gewichtsprozent.

Wird im Rahmen der vorliegenden Erfindung auf die Bezeichnung Gemisch Bezug genommen, so ist hierbei stets eine Mischung zu verstehen, die durch Mischen zuvor klar definierter Komponenten erhalten wurde. Entsprechend sind beispielsweise als Hilfsstoffgemisch aus gröberen und feineren Hilfsstoffanteilen nur solche Gemische zu verstehen: die durch Mischen einer gröberen Hilfsstoffkomponente mit einer feineren Hilfsstoffkomponente erhalten werden.

Die Hilfsstoffbestandteile können aus dem chemisch gleichen oder aus chemisch verschiedenen Substanzen bestehen, wobei Inhalationspulver, bei denen die Hilfsstoffbestandteile aus der selben chemischen Verbindung bestehen bevorzugt sind.

Wird als Hilfsstoff ein Gemisch aus gröberen und feineren Hilfststofffraktionen verwendet, so können diese ebenfalls aus dem chemisch gleichen oder aus chemisch verschiedenen Substanzen bestehen, wobei Inhalationspulver, bei denen der gröbere Hilfsstoffanteil und der feinere Hilfsstoffanteil aus der selben chemischen Verbindung bestehen bevorzugt sind.

Als'physiologisch unbedenkliche Hilfsstoffe, die zur Darstellung der im Rahmen der erfindungsgemäßen Inhaletten zur Anwendung gelangenden Inhalationspulver Verwendung finden können seien beispielsweise genannt Monosaccharide (z.B. Glucose oder Arabinose), Disaccharide (z.B. Lactose, Saccharose, Maltose), Oligound Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist Als besonders bevorzugt im Sinne der Erfindung gelangt Lactose, höchst bevorzugt Lactosemonohydrat als Hilfsstoff zur Anwendung.

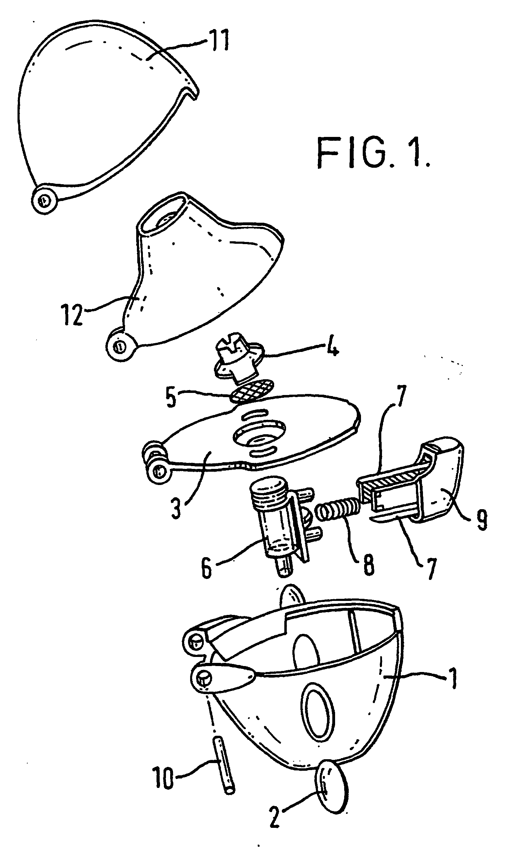

Die erfindungsgemäßen Inhalationskapseln können beispielsweise mittels Inhalatoren appliziert werden, wie sie in der WO 94/28958 beschrieben werden.
Ein zur Anwendung der erfindungsgemäßen Inhaletten besonders bevorzugt einsetzbarer Inhalator ist in Figur 1 als Explosionszeichnung dargestellt.
Dieser Inhalator (HandiHaler) für die Inhalation pulverförmiger Arzneimittel aus Inhalationskapseln ist gekennzeichnet durch ein Gehäuse 1, enthaltend zwei Fenster 2, ein Deck 3, in dem sich Lufteinlaßöffnungen befinden und welches mit der Kapselkammer 6 verbunden ist, an der ein mit zwei geschliffenen Nadeln 7 versehener, gegen eine Feder 8 beweglicher Drücker 9 vorgesehen ist, sowie ein über eine Achse 10 klappbar mit dem Gehäuse 1, dem Deck 3 und einer Kappe 11 verbundenes Mundstück 12. Die Kaplselkammer ist mit einem Filter 5 abgschlossen. Das Filter wird von einem mit dem Mundstück 12 fest verbundenem Filterhalter getragen.

Bei den erfindungsgemäßen Inhalationskapseln bieten sich Füllmengen von 2 bis 50mg, bevorzugt von 4 bis 25mg Inhalationspulver pro Inhalationskapsel an. Diese enthalten dann zwischen 1,2 und 80µg Tiotropium. Bei einer besonders bevorzugten Füllmenge von 4 bis 6 mg Inhalationspulver pro Inhalationskapsel sind zwischen 1,6 und 48µg, bevorzugt zwischen 3,2 und 38,4µg, besonders bevorzugt zwischen 6,4 und 24µg Tiotropium pro lnhalationskapsel enthalten. Ein Gehalt von beispielsweise 18µg Tiotropium entspricht dabei einem Gehalt von etwa 21,7µg Tiotropiumbromid.

Folglich enthalten Inhalationskapseln mit einer Füllmenge von 3 bis 10mg Inhalationspulver erfindungsgemäß bevorzugt zwischen 1,4 und 96,3µg Tiotropiumbromid. Bei einer bevorzugten Füllmenge von 4 bis 6 mg Inhatationspulver pro Inhalationskapsel sind zwischen 1,9 und 57,8µg, bevorzugt zwischen 3,9 und 46,2µg, besonders bevorzugt zwischen 7,7 und 28,9µg Tiotropiumbromid pro Inhalationskapsel enthalten. Ein Gehalt von beispielsweise 21,7µg Tiotropiumbromid entspricht dabei einem Gehalt von etwa 22,5µg Tiotropiumbromid-monohydrat.

Folglich enthalten Inhalationskapseln mit einer Füllmenge von 3 bis 10mg Inhalationspulver bevorzugt zwischen 1,5 und 100µg Tiotropiumbromid-monohydrat. Bei einer bevorzugten Füllmenge von 4 bis 6 mg Inhalationspulver pro Inhalationskapsel sind zwischen 2 und 60µg, bevorzugt zwischen 4 und 48µg, besonders bevorzugt zwischen 8 und 30µg Tiotropiumbromid-monohydrat pro Inhalationskapsel enthalten.

Die erfindungsgemäßen Inhalationskapseln sind entsprechend der der vorliegenden Erfindung zugrunde liegenden Aufgabe gekennzeichnet durch ein hohes Maß an Homogenität im Sinne der Einzetdosierungsgenauigkeit. Diese liegt in einem Bereich von < 8% , bevorzugt < 6% , besonders bevorzugt < 4%.

Die zur Befüllung der erfindungsgemäßen Inhalationskapseln vorzugsweise einsetzbaren Inhalationspulver sind gemäß der nachfolgend beschriebenen Vorgehensweise erhältlich.
Nach Einwaage der Ausgangsmaterialien erfolgt in den Fällen, in denen als Hilfsstoff ein Gemisch aus gröberen und feineren Hilfsstoffanteilen zur Einsatz gelangen soll zunächst die Fertigung der Hilfsstoffmischung. Wird als Hilfsstoff eine bezüglich der mittleren Teilchengröße der Hilfsstoffteilchen einheitliche Fraktion (z.B. 15 - 80µm) verwendet, entfällt dieser erste Schritt.
Anschließend erfolgt die Herstellung der Inhalationspulver aus dem Hilfsstoff, gegebenenfalls der Hilfsstoffmischung und dem Wirkstoff. Die erfindungsgemäßen Inhalationskapseln werden vor Befüllung mit dem Tiotropium-haltigen Inhalationspulver getrocknet, bis der erfindungsgemäß maximal zulässige Grad an TEWS- oder Halogentrockner-Feuchte erreicht ist. Daran schließt sich die Fertigung der pulverhaltigen Inhalationskapseln unter Verwendung von im Stand der Technik bekannten Verfahren an.

Bei den nachstehend beschriebenen Herstellverfahren werden die genannten Komponenten in den Gewichtsanteilen eingesetzt, wie sie in den zuvor beschriebenen Zusamensetzungen der Inhalationspulver beschrieben wurden.

Werden als Hilfsstoff Gemische aus gröberen und feineren Hilfsstoffanteilen verwendet, werden die gröberen und feineren Hitfsstoffanteile in einen geeigneten Mischbehälter eingebracht. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird der gröbere Hilfsstoff vorgelegt und anschließend der feinere Hilfsstoffanteil in den Mischbehälter eingebracht. Bevorzugt erfolgt bei diesem Mischverfahren die Zugabe der beiden Komponenten portionsweise, wobei ein Teil des gröberen Hilfsstoffs zunächst vorgelegt und anschließend abwechselnd feinerer und gröberer Hilfsstoff zugegeben wird. Besonders bevorzugt ist bei der Herstellung der Hilfsstoffmischung das abwechselnde, schichtweise Einsieben der beiden Komponenten. Vorzugsweise erfolgt das Einsieben der beiden Komponenten abwechselnd in je 15 bis 45, besonders bevorzugt in je 20 bis 40 Schichten. Der Mischvorgang der beiden Hilfsstoffe kann bereits während der Zugabe der beiden Komponenten erfolgen. Vorzugsweise wird allerdings erst nach dem schichtweisen Einsieben der beiden Bestandteile gemischt.

Vorstehender Schritt entfällt naturgemäß, wenn eine bezüglich der Partikelgröße einheitliche Hilfsstoffraktion verwendet wird (z.B. mittlere Teilchengröße von 15-80µm).

Anschließend werden Hilfsstoff, gegebenenfalls die Hilfsstoffmischung und der Wirkstoff in einen geeigneten Mischbehälter eingebracht. Der verwendete Wirkstoff weist eine mittlere Teilchengröße von 0,5 bis 10µm, vorzugsweise von 1 bis 6µm, besonders bevorzugt von 2 bis 5µm auf. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird der Hilfsstoff vorgelegt und anschließend der Wirkstoff in den Mischbehälter eingebracht. Bevorzugt erfolgt bei diesem Mischverfahren die Zugabe der beiden Komponenten portionsweise. Besonders bevorzugt ist im Falle der Verwendung von Hilfsstoffmischung das abwechselnde, schichtweise Einsieben der beiden Komponenten. Vorzugsweise erfolgt das Einsieben der beiden Komponenten abwechselnd in je 25 bis 65, besonders bevorzugt in je 30 bis 60 Schichten. Der Mischvorgang der Hilfsstoffmischung mit dem Wirkstoff kann bereits während der Zugabe der beiden Komponenten erfolgen. Vorzugsweise wird allerdings erst nach dem schichtweisen Einsieben der beiden Bestandteile gemischt.
Die so erhaltene Pulvermischung kann gegebenenfalls erneut ein- oder mehrfach über einen Siebgranulator gegeben und jeweils anschließend einem weiteren Mischvorgang unterworfen werden.

In einer weiteren bevorzugten Ausführungsform der Erfindung, werden die Inhalationskapseln mit dem Tiotropiumbromid-haltigen, nach obigem Verfahren erhältlichen Inhalationspulver befüllt und anschließend dem nachfolgend beschriebenen Trocknungsprozess unterworfen.
Die befüllten Kapselen werden in einer ersten Phase (Trocknungsphase) für einen Zeitraum von 0,5 - -10 Stunden, vorzugsweise 1,5 - 7 Stunden, bevorzugt 2 - 5,5 Stunden, besonders bevorzugt etwa 2,5 - 4,5 Stunden bei einer Temperatur von etwa 10 - 50°C, bevorzugt 20 - 40 °C, besonders bevorzugt etwa 25 - 35°C einer relativen Feuchte von höchstens 30% r.F., bevorzugt höchstens 20% r.F., besonders bevorzugt von etwa 5 - 15% r.F. ausgesetzt. Unter relativer Feuchte (r.F.) wird im Rahmen der vorliegenden Erfindung der Quotient des Partialdruckes des Wasserdampfs und des Dampfdruckes des Wassers bei der betreffenden Temperatur verstanden. In einer sich daran anschließenden zweiten Phase (Gleichgewichtsphase) werden die Kapseln für einen Zeitraum von 0,5 - 10 Stunden, vorzugsweise 1,5 - 7 Stunden, bevorzugt 2 - 5,5 Stunden, besonders bevorzugt etwa 2,5 - 4,5 Stunden bei einer Temperatur von etwa 10 - 50°C, bevorzugt 20 - 40 °C, besonders bevorzugt etwa 25 - 35°C einer relativen Feuchte von höchstens 35% r.F., bevorzugt höchstens 25% r.F., besonders bevorzugt vo etwa 10 - 20% r.F. ausgesetzt. Hieran schließt sich, sofern die Temperatur in den vorstehenden Verfahrensschritten auf Werte oberhalb der Raumtemperatur (i.e. 23°C) eingestellt wurde, gegebenenfalls eine Abkühlungsphase an. In dieser Abkühlungsphase werden die Kapseln für einen Zeitraum von 0,1 - 6 Stunden, vorzugsweise 0,5 - 4 . Stunden, bevorzugt 0,75 - 2,5 Stunden, besonders bevorzugt etwa 1 - 2 Stunden bei einer Temperatur von etwa 23°C einer relativen Feuchte von höchstens 35% r.F., bevorzugt höchstens 25% r.F., besonders bevorzugt vo etwa 10 - 20% r.F. ausgesetzt. In einer besonders bevorzugten Ausführungsform sind die für die Gleichgewichtsphase und Abkühlungsphase für die relative Feuchte eingestellten Werte identisch.

Wird im Rahmen der vorliegenden Erfindung der Begriff Wirkstoff verwendet, so ist dies als Bezugnahme auf Tiotropium zu verstehen. Eine Bezugnahme auf Tiotropium, welches das freie Ammoniumkation darstellt, entspricht erfindungsgemäß einer Bezugnahme auf Tiotropium in Form eines Salzes (Tiotropium-Salz), welches ein Anion als Gegenion enthält. Als im Rahmen der vorliegenden Erfindung einsetzbare Tiotropium-Salze sind die Verbindungen zu verstehen, die neben Tiotropium als Gegenion (Anion) Chlorid, Bromid, iodid, Methansulfonat, para-Toluolsulfonat oder Methylsulfat enthalten. Im Rahmen der vorliegenden Erfindung ist von allen Tiotropiumsalzen das Tiotropiumbromid bevorzugt. Bezugnahmen auf Tiotropiumbromid sind im Rahmen der vorliegenden Erfindung stets als Bezugnahmen auf alle möglichen amorphen und kristallinen Modifikationen des Tiotropiumbromids zu verstehen. Diese können beispielsweise in der kristallinen Struktur Lösemittelmoleküte mit einschließen. Von allen kristallinen Modifikationen des Tiotropiumbromids sind erfindungsgemäß diejenigen, die Wasser mit einschließen (Hydrate) bevorzugt. Besonders bevorzugt ist im Rahmen der vorliegenden Erfindung das gemäß nachstehend detailliert erläuterter Vorgehensweise erhältliche kristalline Tiotropiumbromid-monohydrat einsetzbar.

Für die Herstellung der erfindungsgemäßen Tiotropium-haltigen Inhalationskapseln ist es zunächst erforderlich, Tiotropium in für die pharmazeutische Anwendung verwendbarer Form bereitzustellen. Bevorzugt wird dazu Tiotropiumbromid, welches wie in der EP 418 716 A1 offenbart, hergestellt werden kann, einem weiteren Kristallisationsschritt unterworfen. Je nach Wahl der Reaktionsbedingungen und des Lösemittels werden dabei unterschiedliche Kristaltmodtfikationen erhalten.
Für die Zwecke der Herstellung der erfindungsgemäßen Inhalationskapseln hat sich das kristalline Tiotropiumbromid-monohydrat als besonders geeignet erwiesen.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### Ausgangsmaterialien

In den nachfolgenden Beispielen wird als Hilfsstoff Lactose-Monohydrat (200M) verwendet. Dieser kann beispielsweise von der Firma DMV International, 5460 Veghel/NL unter der Produktbezeichnung Pharmatose 200M bezogen werden.

In den nachfolgenden Beispielen wird Lactose-Monohydrat (200M) ebenfalls als gröberer Hilfsstoff verwendet, wenn Hilfsstoffmischungen zum Einsatz gelangen. Diese kann beispielsweise von der Firma DMV international, 5460 Veghel/NL unter der Produktbezeichnung Pharmatose 200M bezogen werden.

In den nachfolgenden Beispielen wird im Falle der Verwendung von Hilfsstoffmischungen als feinerer Hilfsstoff Lactose-Monohydrat (5µ) eingesetzt. Diese kann durch gängige Verfahren (Mikronisieren) aus Lactose-Monohydrat 200M erhalten werden. Lactose-Monohydrat 200M kann beispielsweise von der Firma DMV international, 5460 Veghel/NL unter der Produktbezeichnung Pharmatose 200M bezogen werden.

### Darstellung von kristallinem Tiotroniumbromid-monohydrat

In einem geeigneten Reaktionsgefäß werden in 25,7 kg Wasser 15,0-kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet. Ausbeute: 13,4 kg Tiotropiumbromid-monohydrat (86 % d. Th.)

Das gemäß vorstehend beschriebener Vorgehensweise erhältliche kristalline Tiotropiumbromid-monohydrat wurde einer Untersuchung mittels DSC (Differential Scanning Calorimetry) unterworfen. Das DSC- diagramm weist zwei charakteristische Signale auf. Das erste, relativ breite, endotherme Signal zwischen 50-120°C ist auf die Entwässerung des Tiotropiumbomid-monohydrats zur wasserfreien Form zurückzuführen. Das zweite, relativ scharfe, endotherme Maximum bei 230 ± 5°C ist dem Schmelzen der Substanz zuzuordnen. Diese Daten wurden mittels eines Mettler DSC 821 erhalten und mit dem Mettler Software-Paket STAR ausgewertet. Die Daten wurden bei einer Heizrate von 10 K/min erhoben.

Das kristalline Tiotropiumbromid-monohydrat wurde mittels IR-Spektroskopie charakterisiert. Die Daten wurden mittels eines Nicolet FTIR Spektrometers erhoben und mit dem Nicolet Softwarepaket OMNIC, Version 3.1 ausgewertet. Die Messung wurde mit 2,5µmol Tiotropiumbromid-monohydrat in 300 mg KBr durchgeführt.

Die nachstehende Tabelle faßt einige der wesentlichen Banden des IR-Spektrums zusammen.

| Wellenzahl (cm⁻¹) | Zuordnung | Schwingungstyp |
|---|---|---|
| 3570,3410 | O-H | Streckschwingung |
| 3105 | Aryl C-H | Streckschwingung |
| 1730 | C=O | Streckschwingung |
| 1260 | Epoxid C-O | Streckschwingung |
| 1035 | Ester C-OC | Streckschwingung |
| 720 | Thiophen | Ringschwingung |

Das nach vorstehendem Verfahren erhältliche kristalline Tiotropiumbromid-monohydrat weist gemäß Einkristallröntgenstrukturanalyse eine einfache monoklinische Zelle mit folgenden Dimensionen auf: a = 18.0774 Å, b = 11.9711 Å, c = 9.9321 Å, β = 102.691°, V = 2096.96 Å³. Diese Daten wurden mittels eines AFC7R- 4-Kreisdiffraktometer (Rigaku) unter Verwendung von monochromatisierter Kupfer Kα-Strahlung erhoben. Die Strukturlösung und Verfeinerung der Kristallstruktur erfolgte mittels direkter Methoden (Programm SHELXS86) und FMLQ-Vefeinerung (Programm TeXsan).

Das so erhaltene kristalline Tiotropiumbromid-monohydrat wird nach bekannten Verfahren mikronisiert, um den Wirkstoff in Form der mittleren Teilchengröße bereitzustellen, die den erfindungsgemäßen Spezifikationen entspricht.

Nachfolgend wird beschrieben, wie die Bestimmung der mittleren Teilchengröße der verschiedenen Bestandteile der erfindungsgemäßen Formulierung erfolgen kann.

### A) Partikelgrößenbestimmung von feinteiliger Lactose:

### Meßgerät und Einstellungen:

Die Bedienung der Geräte erfolgt in Übereinstimmung mit den Bedienungsanleitungen des Herstellers.
- Meßgerät:: HELOS Laser-Beugungs-Spektrometer, (SympaTec)
- Dispergiereinheit:: RODOS Trockendispergierer mit Saugtrichter, (SympaTec)
- Probenmenge:: ab 100 mg
- Produktzufuhr:: Schwingrinne Vibri, Fa. Sympatec
- Frequenz d. Vibrationsrinne:: 40 bis 100 % ansteigend
- Dauer der Probenzufuhr:: 1 bis 15 sek. (im Fall von 100 mg)
- Brennweite:: 100 mm (Meßbereich: 0,9 -175 µm)
- Meßzeit:: ca. 15 s (im Fall von 100 mg)
- Zykluszeit:: 20 ms
- Start/Stop bei:: 1 % auf Kanal 28
- Dispergiergas:: Druckluft
- Druck:: 3 bar
- Unterdruck:: maximal
- Auswertemodus:: HRLD

### Probenvorbereitung / Produktzufuhr:

Mind. 100 mg der Prüfsubstanz werden auf einem Kartenblatt eingewogen. Mit einem weiteren Kartenblatt werden alle größeren Agglomerate zerstoßen. Das Pulver wird dann auf der vorderen Hälfte der Schwingrinne (ab ca. 1 cm vom vorderen Rand) fein verteilt aufgestreut. Nach dem Start der Messung wird die Frequenz der Schwingrinne von ca. 40 % bis 100 % (gegen Ende der Messung) variiert. Die Zeit, in der jeweils die gesamte Probe zugeführt wird beträgt 10 bis 15 sek.

### B) Partikelgrößenbestimmung von Tiotropium Monohydrat, mikronisiert:

### Meßgerät und Einstellungen:

Die Bedienung der Geräte erfolgte in Übereinstimmung mit den Bedienungsanleitungen des Herstellers.
- Meßgerät:: Laser-Beugungs-Spektrometer (HELOS), Sympatec
- Dispergiereinheit:: Trockendispergierer RODOS mit Saugtrichter,Sympatec
- Probenmenge:: 50 mg - 400 mg
- Produktzufuhr:: Schwingrinne Vibri, Fa. Sympatec
- Frequenz d. Vibrationsrinne:: 40 bis 100 % ansteigend
- Dauer der Probenzufuhr:: 15 bis 25 sek. (im Fall von 200 mg)
- Brennweite:: 100 mm (Meßbereich: 0,9 -175 µm)
- Meßzeit:: ca. 15 s . (im Fall von 200 mg)
- Zykluszeit:: 20 ms
- Start/Stop bei:: 1 % auf Kanal 28
- Dispergiergas:: Druckluft
- Druck:: 3 bar
- Unterdruck:: maximal
- Auswertemodus:: HRLD

### Probenvorbereitung/Produktzufuhr:

ca. 200 mg der Prüfsubstanz werden auf einem Kartenblatt eingewogen. Mit einem weiteren Kartenblatt werden alle größeren Agglomerate zerstoßen.
Das Pulver wird dann auf der vorderen Hälfte der Schwingrinne (ab ca. 1 cm vom vorderen Rand) fein verteilt aufgestreut.
Nach dem Start der Messung wird die Frequenz der Schwingrinne von ca. 40 % bis. 100 % (gegen Ende der Messung) variiert. Die Zufuhr der Probe soll möglicht kontinuierlich sein. Die Produktmenge darf aber auch nicht zu groß sein damit eine ausreichende Dispergierung erreicht wird. Die Zeit, in der jeweils die gesamte Probe zugeführt wird beträgt für 200 mg z. B. ca. 15 bis 25 sek.

### C) Partikelgrößenbestimmung von Laktose 200M :

### Meßegrät und Einstellungen

Die Bedienung der Geräte erfolgte in Übereinstimmung mit den Bedienungsanleitungen des Herstellers.
- Meßgerät:: Laser-Beugungs-Spektrometer (HELOS), Sympatec
- Dispergiereinheit:: Trockendispergierer RODOS mit Saugtrichter, Sympatec
- Probenmenge:: 500 mg
- Produktzufuhr:: Vibrationsrinne Typ VIBRI , Sympatec
- Frequenz d. Vibrationsrinne:: 18 bis 100 % ansteigend
- Brennweite (1):: 200 mm (Meßbereich: 1.8 - 350 µm)
- Brennweite (2):: 500 mm (Meßbereich: 4.5 - 875 µm)
- Meßzeit/Wartezeit:: 10 s
- Zykluszeit:: 10 ms
- Start/Stop bei:: 1 % auf Kanal 19
- Druck:: 3 bar
- Unterdruck:: maximal
- Auswertemodus:: HRLD

### Probenvorbereitung / Produktzufuhr:

Ca. 500 mg der Prüfsubstanz werden auf einem Kartenblatt eingewogen. Mit einem weiteren Kartenblatt werden alle größeren Agglomerate zerstoßen. Das Pulver wird in der Trichter der Vibrationsrinne überführt Es wird ein Abstand von 1.2 bis 1.4 mm zwischen Vibrationsrinne und Trichter eingestellt Nach dem Start der Messung wird die Amplitudeneinstellung der Schwingrinne von 0 auf 40 % gesteigert bis sich ein kontinuierlicher Produktfluß einstellt. Danach wird auf eine Amplitude von ca. 18% reduziert. Gegen Ende der Messung wird die Amplitude auf 100% gesteigert.

### Apparatives

Zur Herstellung der Inhalationspulver können beispielsweise die folgenden Maschinen und Geräte Verwendung finden:
Mischbehälter bzw. Pulvermischer: Rhönradmischer 200 L; Typ: DFW80N-4; Hersteller: Firma Engelsmann, D-67059 Ludwigshafen.
Siebgranulator: Quadro Comil; Typ: 197-S; Hersteller: Firma Joisten & Kettenbaum, D-51429 Bergisch-Gladbach.

Zur Bestimmung der TEWS-Feuchte wird das nachstehende Gerät unter Anwendung der hersteflerseits erfolgenden Anwendungsbeschreibung verwendet.

### Gerät zur Bestimmung der TEWS-Feuchte:

Hersteller: Firma TEWS Elektronik, Hamburg
Typ: MW 2200
Meßbereich: 1 bis 85% Feuchtigkeit
Meßgenauigkeit: 1 % vom Endwert des gewählten Meßbereichs.
Netzanschluß: 220 V +/- 10%, 50 - 60 Hz

Zur Bestimmung der Halogentrockner-Feuchte sowie zur Eignung des TEWS-Geräts wird das nachstehende Gerät unter Anwendung der herstellerseits erfolgenden Anwendungsbeschreibung verwendet.

### Gerät zur Besfimmung der Halogentrockner-Feuchte:

Mettler Halogentrockner HR 73; Hersteller: Firma Mettler-Toledo, D-35396 Gießen;

Zur Befüllung der leeren Inhaltionskapseln mittels Tiotropium-haltigen Inhaltionspulver wird das nachstehende Gerät verwendet.

### Kapselfüllmaschine:

MG2, Typ G100, Hersteller: MG2 S.r.l, I-40065 Pian di Macina di Pianoro (BO), Italien;

### Beispiel 1:

### 1.1: Hilfsstoffmischung:

Als gröbere Hilfsstoffkomponente werden 31,82 kg Lactose Monohydrat für Inhalationszwecke (200M) eingesetzt. Als feinere Hilfsstoffkomponente werden 1,68 kg Lactose Monohydrat (5µm) eingesetzt. In den daraus erhaltenen 33,5 kg Hilfsstoffmischung beträgt der Anteil der feineren Hilfsstoffkomponente 5%.

Ober einen geeigneten Siebgranulator mit einem Sieb mit einer Maschenweite von 0,5 mm werden in einen geeigneten Mischbehälter ca. 0,8 bis 1,2 kg Lactose Monohydrat für Inhalationszwecke (200M) vorgelegt. Anschließend werden abwechselnd Lactose Monohydrat (5µm) in Portionen von ca. 0,05 bis 0,07 kg und Lactose Monohydrat für inhalationszwecke (200M) in Portionen von 0,8 bis 1,2 kg schichtweise eingesiebt. Lactose Monohydrat für Inhalationszwecke (200M) und Lactose Monohydrat (5µm) werden in 31 bzw. in 30 Schichten (Toleranz: ±6 Schichten) zugegeben.

Die eingesiebten Bestandteile werden anschließend gemischt (Mischen: 900 Umdrehungen).

### 1.2: Endmischung:

Zur Herstellung der Endmischung werden 32,87 kg der Hilfsstoffmischung (1.1) und 0,13 kg mikronisiertes Tiotropiumbromid-monohydrat eingesetzt. In den daraus erhaltenen 33,0 kg Inhalationspulver beträgt der Wirkstoffanteil 0.4%.

Entsprechend wird unter alleiniger Verwendung von 32,87kg Lactose-Monohydrat (200M) verfahren, wenn als Hilfsstoff eine bezüglich der mittleren Teilchengröße einheitliche Hilfsstofffraktion zum Einsatz gelangt. In diesem Fall wird naturgemäß auf die Durchführung des Schrittes 1.1 verzichtet.

Über einen geeigneten Siebgranulator mit einem Sieb mit einer Maschenweite von 0,5 mm werden in einen geeigneten Mischbehälter ca. 1,1 bis 1,7 kg Hilfsstoff oder Hilfsstoffmischung (1.1) vorgelegt. Anschließend werden abwechselnd Tiotropiumbromid-monohydrat in Portionen von ca. 0,003 kg und Hilfsstoff oder Hilfsstoffmischung (1.1) in Portionen von 0,6 bis 0,8 kg schichtweise eingesiebt. Die Zugabe des Hilfsstoffs oder der Hilfststoffmischung und des Wirkstoffs erfolgt in 47 bzw. 45 Schichten (Toleranz: ± 9 Schichten).

Die eingesiebten Bestandteile werden anschließend gemischt (Mischen: 900 Umdrehungen). Die Endmischung wird noch zweimal über einen Siebgranulator gegeben und anschließend jeweils gemischt (Mischen: 900 Umdrehungen).

### Beispiel 2:

Mit der nach Beispiel 1 erhaltenen Mischung werden Inhalationskapseln (Inhaletten) der folgenden Zusammensetzung erhalten:

| | |
|---|---|
| Tiotropiumbromid-monohydrat | 0,0225 mg |
| Lactose Monohydrat (200 M) | 5,2025 mg |
| Lactose Monohydrat (5 µm) | 0,2750 mg |
| Hartgelatinekapsel (5% PEG 3350; 9%-TEWS-Feuchte) | 49,0 mg |
| Total | 54,5 mg |

### Beispiel 3:

### Inhalationskapsel:

| | |
|---|---|
| Tiotropiumbromid-monohydrat | 0,0225 mg |
| Lactose Monohydrat (200 M) | 4,9275 mg |
| Lactose Monohydrat (5 µm) | 0,5500 mg |
| Hartgelatinekapsel (5% PEG 3350: 9%-TEWS-Feuchte) | 49,0 mg |
| Total | 54,5 mg |

Das zur Herstellung der lnhalationskapsel erforderliche Inhalationspulver wurde in Analogie zu Beispiel 1 erhalten.

### Beispiel 4:

### Inhalationskapsel:

| | |
|---|---|
| Tiotropiumbromid-monohydrat | 0,0225 mg |
| Lactose Monohydrat (200 M) | 5,2025 mg |
| Lactose Monohydrat (5 µm) | 0,2750 mg |
| HPMC (<2% TEWS-Feuchte) | 49,0 mg |
| Total | 54,5 mg |

Das zur Herstellung der Inhalationskapsel erforderliche Inhalationspulver wurde in Analogie zu Beispiel 1 erhalten.

### Beispiel 5:

### Inhalationskapsel:

| | |
|---|---|
| Tiotropiumbromid-monohydrat | 0,0225 mg |
| Lactose Monohydrat (200 M) | 5,2025 mg |
| Lactose Monohydrat (5 µm) | 0,2750 mg |
| Polyethvlen (<1% TEWS-Feuchte) | 100.0 mg |
| Total | 105,5 mg |

Das zur Herstellung der Inhalationskapsel erforderliche Inhalationspulver wurde in Analogie zu Beispiel 1 erhalten.

### Beispiel 6:

### Inhalationskapsel:

| | |
|---|---|
| Tiotropiumbromid-monohydrat | 0,0225 mg |
| Lactose Monohydrat (200 M) | 5,4775 mg |
| Polyethylen (<1% TEWS-Feuchte) | 100,0 mg |
| Total | 105,5 mg |

Das zur Herstellung der Inhalationskapsel erforderliche Inhalationspulver wurde in Analogie zu Beispiel 1 erhalten.

### Beispiel 7:

Mit der nach Beispiel 1 erhaltenen Mischung werden Inhalationskapseln (Inhaletten) der folgenden Zusammensetzung erhalten:

| | |
|---|---|
| Tiotropiumbromid-monohydrat | 0,0225 mg |
| Lactose Monohydrat (200 M) | 5,2025 mg |
| Lactose Monohydrat (5 µm) | 0,2750 mg |
| Hartgelatinekapsel (5% PEG 3350) | 49,0 mg |
| Total | 54,5 mg |

Diese Kapseln werden unter geeigneten Klimabedingungen in einer Klimakammer auf einen Wassergehalt von ca. 8.7 % (Messung mit Mikrowellenfeuchtemeßgerät TEWS) gemäß nachfolgender Vorgehensweise eingestellt. Zu Beginn wird eine Trocknungsphase durchgeführt, der sich eine sogenannte Gleichgewichtsphase anschließt. Schließlich werden die Kapseln einer sogenannten Abkühlphase unterworfen. Die so getrockneten Kapseln werden direkt im Anschluß in entsprechende lagerstabile Vorratsgebinde oder ähnliches verpackt.

### Prozeßdaten

Einstellungen der Klimabedingungen auf folgende Sollwerte:

| | |
|---|---|
| Trocknungsphase | 30 °C |
| | 10 % r.F. (relative Feuchte) |
| | 3.5 h |
| Gleichgewichtsphase | 30 °C |
| | 16 % r.F. |
| | 3.5 h |
| Abkühlphase | 23 °C |
| | 16 % r.F. |
| | 1.5 h |

Unter relativer Feuchte (r.F.) wird im Rahmen der vorliegenden Erfindung der Quotient des Partialdruckes des Wasserdampfs und des Dampfdruckes des Wassers bei der betreffenden Temperatur verstanden.

Im Sinne der vorliegenden Erfindung ist unter mittlerer Teilchengröße der Wert in µm zu verstehen, an dem 50% der Teilchen aus der Volumenverteilung eine kleinere oder die gleiche Partikelgröße besitzen im Vergleich zum angegebenen Wert. Zur Bestimmung der Summenverteilung der Partikelgrößenverteilung wird als Meßmethode Laserdiffraktion/Trockendispergierung angewendet.

## Patentansprüche

1. Inhalationskapseln, die als Inhalationspulver Tiotropium im Gemisch mit einem physiologisch unbedenklichen Hilfsstoff enthalten, **dadurch gekennzeichnet, daß** das Kapselmaterial einen reduzierten Feuchtegehalt als TEWS-oder Halogentrockner-Feuchte von weniger als 15%) aufweist.

2. Inhalationskapseln nach Anspruch 1, **dadurch gekennzeichnet, daß** das Kapselmaterial ausgewählt ist aus der Gruppe bestehend aus Gelatine, Cellulosederivaten, Stärke, Stärkederivaten, Chitosan und synthetischen Kunststoffen.

3. Inhalationskapseln nach Anspruch 2, **dadurch gekennzeichnet, daß** als Kapselmaterial Gelatine im Gemisch mit anderen Zusätzen ausgewählt aus der Gruppe bestehend aus Polyethylenglycol (PEG), bevorzugt PEG 3350, Glycerol, Sorbitol, Propylenglycol, PEO-PPO-Blockcopolymeren und anderen Polyalkoholen und Polyethern verwendet wird.

4. Inhalationskapseln nach Anspruch 3, **dadurch gekennzeichnet, daß** das Kapselmaterial neben Gelatine PEG in einem Anteil von 1-10 Gew-%, bevorzugt 3-8 %, enthält.

5. Inhalationskapseln nach Anspruch 3 oder 4, **dadurch gekennzeichnet, daß** das Kapselmaterial eine TEWS- oder Halogentrockner-Feuchte von weniger als 12%, besonders bevorzugt von ≤ 10% aufweist.

6. Inhalationskapseln nach Anspruch 2, **dadurch gekennzeichnet, daß** das Kapselmaterial ausgewählt ist aus der Gruppe der Cellulosederivate Hydroxypropylmethytcellulose, Hydroxypropylcellulose, Methylcellulose, Hydroxymethylcellulose und Hydroxyethylcellulose.

7. Inhalationskapseln nach Anspruch 6, **dadurch gekennzeichnet, daß** das Kapselmaterial eine TEWS- oder Halogentrockner-Feuchte von weniger als 8%, besonders bevorzugt von ≤ 5% aufweist.

8. Inhalationskapseln nach Anspruch 2, **dadurch gekennzeichnet, daß** das Kapselmaterial ausgewählt ist aus der Gruppe der synthetischen Kunststoffe Polyethylen, Polycarbonat, Polyester, Polypropylen und Polyethylenterephthalat.

9. Inhalationskapseln nach Anspruch 8, **dadurch gekennzeichnet, daß** das Kapselmaterial ausgewählt ist aus Polyethylen, Polycarbonat und Polyethylenterephthalat.

10. Inhalationskapseln nach Anspruch 8 oder 9, **dadurch gekennzeichnet, daß** das Kapselmaterial eine TEWS- oder Halogentrockner-Feuchte von weniger als 3%, besonders bevorzugt von ≤ 1% aufweist.

11. Inhalationskapseln nach einem der Ansprüche 1 bis 10, **dadurch gekennzeichnet, daß** das Inhalationspulver 0,001 bis 2% Tiotropium im Gemisch mit einem physiologischen unbedenklichen Hilfsstoff enthält.

12. Inhalationskapseln nach Anspruch 11, **dadurch gekennzeichnet, daß** der Hilfsstoff aus einem Gemisch von gröberem Hilfsstoff mit einer mittleren Teilchengröße von 15 bis 80µm und feinerem Hilfststoff mit einer mittleren Teilchengröße von 1 bis 9 µm besteht, wobei der Anteil von feinerem Hilfsstoff an der Gesamthilfsstoffmenge 1 bis 20% beträgt.

13. Inhalationskapseln nach Anspruch 12, **dadurch gekennzeichnet, daß** das Tiotropium in Form seines Chlorids, Bromids, lodids, Methansulfonats, para-Toluolsulfonats oder Methylsulfats vorliegt.

14. Verwendung von Inhalationskapseln nach einem der Ansprüche 1 bis 13 und eines Inhalators zur Herstellung eines Medikament zur Inhalation.

15. Verwendung nach Anspruch 14 zur Behandlung von Asthma oder COPD.

16. Verwendung von leeren Kapseln die durch eine TEWS- oder Halogentrockner-Feuchte von weniger als 15% **gekennzeichnet** sind, zur Herstellung von Tiotropium-haltigen Inhalationskapseln nach einem der Ansprüche 1 bis 13.

## Claims

1. Capsules for inhalation which contain as the inhalable powder tiotropium in admixture with a physiologically acceptable excipient, **characterised in that** the capsule material has a reduced moisture content as a TEWS or halogen drier moisture content of less than 15%.

2. Capsules for inhalation according to claim 1, **characterised in that** the capsule material is selected from among gelatine, cellulose derivatives, starch, starch derivatives, chitosan and synthetic plastics.

3. Capsules for inhalation according to claim 2, **characterised in that** the capsule material used is gelatine in admixture with other additives selected from among polyethyleneglycol (PEG), preferably PEG 3350, glycerol, sorbitol, propyleneglycol, PEO-PPO block copolymers and other polyalcohols and polyethers.

4. Capsules for inhalation according to claim 3, **characterised in that** the capsule material contains, in addition to gelatine, PEG in an amount of 1-10 wt.-%, preferably 3-8 %.

5. Capsules for inhalation according to claim 3 or 4, **characterised in that** the capsule material has a TEWS or halogen drier moisture content of less than 12%, particularly preferably ≤ 10%.

6. Capsules for inhalation according to claim 2, **characterised in that** the capsule material is selected from the cellulose derivatives hydroxypropylmethylcellulose, hydroxypropylcellulose, methylcellulose, hydroxymethylcellulose and hydroxyethylcellulose.

7. Capsules for inhalation according to claim 6, **characterised in that** the capsule material has a TEWS or halogen drier moisture content of less than 8%, particularly preferably ≤ 5%.

8. Capsules for inhalation according to claim 2, **characterised in that** the capsule material is selected from the synthetic plastics polyethylene, polycarbonate, polyester, polypropylene and polyethylene terephthalate.

9. Capsules for inhalation according to claim 8, **characterised in that** the capsule material is selected from polyethylene, polycarbonate and polyethylene terephthalate.

10. Capsules for inhalation according to claim 8 or 9, **characterised in that** the capsule material has a TEWS or halogen drier moisture content of less than 3%, particularly preferably ≤ 1%.

11. Capsules for inhalation according to one of claims 1 to 10, **characterised in that** the inhalable powder contains 0.001 to 2% tiotropium in admixture with a physiologically acceptable excipient.

12. Capsules for inhalation according to claim 11, **characterised in that** the excipient consists of a mixture of coarser excipient with an average particle size of 15 to 80*µ*m and finer excipient with an average particle size of 1 to 9 *µ*m, the proportion of finer excipient in the total quantity of excipient being 1 to 20%.

13. Capsules for inhalation according to claim 12, **characterised in that** the tiotropium is in the form of its chloride, bromide, iodide, methanesulphonate, para-toluenesulphonate or methylsulphate.

14. Use of capsules for inhalation according to one of claims 1 to 13 and an inhaler for preparing a medicament for inhalation.

15. Use according to claim 14 for the treatment of asthma or COPD.

16. Use of empty capsules which are **characterised by** a TEWS or halogen drier moisture content of less than 15%, for preparing tiotropium-containing capsules for inhalation according to one of claims 1 to 13.

## Revendications

1. Capsules pour inhalation qui contiennent comme poudre pour inhalation du tiotropium en mélange avec un adjuvant physiologiquement acceptable, **caractérisées en ce que** la matière des capsules présente une teneur en humidité réduite, en humidité TEWS ou au sécheur à halogène, inférieure à 15 %.

2. Capsules pour inhalation selon la revendication 1 **caractérisées en ce que** la matière des capsules est choisie dans le groupe consistant en la gélatine, les dérivés cellulosiques, l'amidon, les dérivés de l'amidon, le chitosane et les matières plastiques synthétiques.

3. Capsules pour inhalation selon la revendication 2 **caractérisées en ce que** la gélatine en mélange avec d'autres additifs choisis dans le groupe consistant en le polyéthylèneglycol (PEG), de préférence le PEG 3350, le glycérol, le sorbitol, le propylèneglycol, les copolymères séquencés POE-POP et d'autres polyalcools et polyéthers est utilisée comme matière des capsules.

4. Capsules pour inhalation selon la revendication 3 **caractérisées en ce que** la matière des capsules contient, outre la gélatine, du PEG en une proportion de 1-10 % en masse, de préférence 3-8 %.

5. Capsules pour inhalation selon la revendication 3 ou 4 **caractérisées en ce que** la matière des capsules présente une humidité TEWS ou au sécheur à halogène inférieure à 12 %, de manière particulièrement préférée ≤10 %.

6. Capsules pour inhalation selon la revendication 2 **caractérisées en ce que** la matière des capsules est choisie dans le groupe des dérivés cellulosiques hydroxypropylméthylcellulose, hydroxypropylcellulose, méthylcellulose, hydroxyméthylcellulose et hydroxyéthylcellulose.

7. Capsules pour inhalation selon la revendication 6 **caractérisées en ce que** la matière des capsules présente une humidité TEWS ou au sécheur à halogène inférieure à 8 %, de manière particulièrement préférée ≤ 5 %.

8. Capsules pour inhalation selon la revendication 2 **caractérisées en ce que** la matière des capsules est choisie dans le groupe des matières plastiques synthétiques polyéthylène, polycarbonate, polyester, polypropylène et polyéthylènetéréphtalate.

9. Capsules pour inhalation selon la revendication 8 **caractérisées en ce que** la matière des capsules est choisie parmi le polyéthylène, le polycarbonate et le polyéthylènetéréphtalate.

10. Capsules pour inhalation selon la revendication 8 ou 9 **caractérisées en ce que** la matière des capsules présente une humidité TEWS ou au sécheur à halogène inférieure à 3 %, de manière particulièrement préférée ≤ 1 %.

11. Capsules pour inhalation selon l'une des revendications 1 à 10 **caractérisées en ce que** la poudre pour inhalation contient 0,001 à 2 % de tiotropium en mélange avec un adjuvant physiologiquement acceptable.

12. Capsules pour inhalation selon la revendication 11 **caractérisées en ce que** l'adjuvant consiste en un mélange d'adjuvant relativement grossier ayant une taille de particule moyenne de 15 à 80 *µ*m et d'adjuvant relativement fin ayant une taille de particule moyenne de 1 à 9 *µ*m, où la proportion d'adjuvant relativement fin par rapport à la quantité totale d'adjuvant est 1 à 20 %.

13. Capsules pour inhalation selon la revendication 12 **caractérisées en ce que** le tiotropium est sous forme de son chlorure, bromure, iodure, méthanesulfonate, paratoluènesulfonate ou méthylsulfate.

14. Utilisation de capsules pour inhalation selon l'une des revendications 1 à 13 et d'un inhalateur pour la production d'un médicament pour l'inhalation.

15. Utilisation selon la revendication 14 pour le traitement de l'asthme ou de la COPD.

16. Utilisation de capsules vides qui sont **caractérisées par** une humidité TEWS ou au sécheur à halogène inférieure à 15 % pour la production de capsules pour inhalation contenant du tiotropium selon l'une des revendications 1 à 13.
